# EUROPÄISCHE PATENTANMELDUNG

(11) **EP 0 775 744 A2**
(43) Veröffentlichungstag der Anmeldung: **28.05.1997**
(21) Anmeldenummer: 96114992.9
(22) Anmeldetag: 19.09.1996
(51) Int. Cl.: C12N 15/00

(54) **Verwendung von Bor zur Regulierung der genetischen Transposition**

(30) Priorität: 24.11.1995 DE 19543898
(71) Anmelder: GSF-Forschungszentrum für Umwelt und Gesundheit, GmbH, D-85764 Oberschleissheim (DE)
(72) Erfinder: Bengsch, Eberhard, Prof. Dr., 81549 München (DE); Polster, Jürgen, Prof. Dr., 85356 Freising (DE); Forkmann, Gut, Prof. Dr., 85356 Freising (DE); Kettrup, Antonius, Prof. Dr., 59821 Arnsberg (DE)

(57) **Zusammenfassung**

Die Erfindung betrifft die Verwendung von Bor in Form von bioassimilierbaren Borverbindungen.

Aufgabe der Erfindung ist es, die Transposition in einen effizienten, technisch verwertbaren Prozeß überzuführen.

Gelöst wird diese Aufgabe durch die Verwendung von Bor zur Regulierung der genetischen Transposition bei Pflanzen, Tieren, deren Zellkulturen und bei Mikroorganismen.

## Beschreibung

Die Erfindung betrifft die Verwendung von Bor.

Normalerweise sind genetische Transpositionsereignisse in den Genomen von Pflanzen und Tieren (sowie deren Zellkulturen) und von Mikroorganismen extrem seltenen.

Die Transposition ist natürlicherweise ineffizient und technisch nicht anwendbar.

Transponierbare Elemente sind Nukleinsäureabschnitte, die innerhalb der perlschnurartig festliegenden Reihenfolge der Gene im Genom einer Spezies ihre Position wechseln können. Sie lösen sich aus Donorstellen und werden zu mehr oder weniger weit entfernten Zielorten in der DNA (kodierende und regulatorische Einheiten, Introns und andere nichtkodierende Bereiche) transponiert.

Dadurch wird die ursprüngliche Reihenfolge von Genen verändert. Es kommt zur Aktivierung und Inaktivierung von Genen. Gelegentlich treten als makroskopisch sichtbare Zeichen Chromosomenbrüche auf.

Als Gesamteffekt resultiert daraus eine Auflockerung der chromosomalen Ordnung. Die neue Merkmalsausbildung zeigt eine Verteilung, die mit der klassischen Genetik nicht in Einklang zu bringen ist.

Dies führte bereits 1947 (B. McClintock, Carnegie Inst. Washington - Year Book 46 (1947) 146 - 152) zur Entdeckung der Transposonen als damals noch hypothetische springende Gene: Unregelmäßige Pigmentierungsmuster an Maiskörnern (Bezirke mit oder ohne Anthocyansynthese) waren mit den Mendelschen Gesetzen unvereinbar. Hingegen waren sie beispielsweise mit mikroskopisch sichtbaren Chromosomenbrüchen an spezifischen Sollbruchstellen streng korreliert (Ac/Ds-System). Ohne Kenntnis der genetischen Details wurden transpositionsbedingte Mutationen bereits viel früher von Humboldt und Darwin beschrieben.

Seit dem Start von 1947 durch McClintock hat sich die Untersuchung der genetischen Transposition zu einem weitreichenden beschreibenden Wissenszweig entwickelt.

Den vielfältigen Anwendungsmöglichkeiten dieses Wissenspotentials stellte sich bisher ein grundsätzliches Hindernis blockierend entgegen: die extreme Seltenheit der transpositionalen Ereignisse in natürlichen Prozessen und die daraus resultierende quasi-totale Ineffizienz der Transposition.

Für Laborversuche, insbesondere für die Lokalisierung und Klonierung von Genen ("gene tagging/transposon tagging") und für deren wirtschaftlichen Anwendungen ist ein im Evolutionstempo ablaufender Prozeß (ca. 1 - 2 % natürliche Variation der DNA in der Gesamt-Erbinformation der Biosphäre pro 1 Mio. Jahre) zu langsam und zu aufwendig.

Es mangelte bisher nicht an Versuchen, die Transposonen durch physikalische, chemische und biologische Einwirkung in Bewegung zu bringen: Radioaktive, Röntgen-, und UV-Bestrahlung, chemische Zellgifte und virale Infektionen erhöhen die Transpositionsrate und können zur Sichtbarmachung des Phänomens dienen. So waren die radioaktiven Niederschläge aus amerikanischen Kernwaffenexplosionen die Auslöser für das Erscheinen instabiler Pigmentierungsmuster am Maiskorn (En/I bzw. Spm System) und von ebenfalls transposonenbedingten Chlorophylldefekten in Blättern.

Jedoch ist die Verwendung dieser Mittel zur Transposonenaktivierung von schweren degenerativen Veränderungen der Organismen begleitet, was ein Monitoring und die gezielte systematische Verwendung wesentlich erschwert.

Dies wird durch die Behandlung der Organismen mit anorganischen und organischen Borverbindungen erreicht.

Weiterhin ist ein erfolgreicher Einsatz von Transposonen z. B. als genetische Werkzeuge ("transposon tagging") ebenfalls nur durch eine signifikante Erhöhung der Transpositionsrate wirtschaftlich.

Aufgabe der Erfindung ist es, die Transposition in einen effizienten, technisch verwertbaren Prozeß überzuführen.

Gelöst wird diese Aufgabe durch die Verwendung von Bor gemäß Patentanspruch 1. Die Unteransprüche beschreiben vorteilhafte Ausgestaltungen der Erfindung.

Besondere Vorteile der Erfindung sind:
- drastisch verbesserte Effizienz bei der gezielten Suche nach einzelnen Genen in nichtsequenzierten Genomen mit Hilfe von Transposonen ("gene tagging")
- verstärkte transposonen-induzierte Mutation mit anschließender Selektion gewünschter Mutanten: beschleunigte Züchtungserfolge
- beschleunigte Entfernung funktionshemmender Transposonen aus den Genomen von Sorten und Arten zwecks Aktivierung von Genen für wirtschaftlich interessante Merkmale, z. B. zur Erhöhung ihrer Resistenz gegen chemische und biologische Noxen und gegen Infektionen.
- Gezielter Gentransfer durch verstärkt mobilisierte transponierbare Elemente; beispielsweise selektive Inhibierung viraler Zyklen und tumoraler Zellproliferationen.

Es wurde festgestellt, daß überraschenderweise durch Applikation von bio-assimilierbaren Borverbindungen die Excisionsrate von transponierbaren Elementen um Zehnerpotenzen erhöht werden kann, ohne daß es zu zellzerstörenden Nebeneffekten kommt.

Dadurch rückt die Häufigkeit der Transposition als Prozeß in die Nähe anderer fundamentaler genetischer Grundprozesse, wie
- der artenstabilisierenden allgemeinen Replikation und
- der Mutationen einschließlich der "artenaufweichenden" viralen Aktivitäten,
beides von Natur aus Prozesse von hoher Effizienz.

Wegen der Vielzahl der Anwendungen, die sich aus der erfindungsgemäß erzielten Erhöhung der Transpositionsrate ergeben, ist eine kurze Beschreibung des Standes der Technik notwendig.

### Struktur und Vorkommen von Transposonen

Transposonen weisen eine universelle Standardstruktur auf, die von dem beherbergenden Wirtsgenom und seinen Sequenzen unabhängig ist. Von Wirtsseite ist keinerlei Homologie zwischen Excisionsort, Donor-, Transposonen- und Rezeptor-DNA (Integrationsort) erforderlich.

Transposonen bestehen aus einem Grundkörper, der als Mindestausrüstung die Gene zur Kodierung wichtiger Eigenfunktionsproteine enthält: in jedem Falle Transposase(n), ggf. Resolvase, Integrase, Repressionsproteine. Der kodierende einfache oder komplexe Zentralkörper wird beiderseits endständig von transposonenspezifischen umgekehrten Sequenzwiederholungen flankiert. Mit Hilfe der Sequenzwiederholungen und der Transposase schneiden sich die Transposonen strangversetzt aus dem Ursprungslokus heraus und reintegrieren in Zielgene. Dabei werden in der Regel weniger weit entfernte potentielle Integrationsstellen bevorzugt. 50 % der Transpositionen erfolgen in geringer Entfernung vom Excisionsort, ohne das Centromer des betroffenen Chromosoms zu überschreiten. Die übrigen Transposonen fixieren sich in weiter entfernten Bezirken des Gesamtgenoms.

Transposonen sind ubiquitäre genetische Einheiten, die in der gesamten lebenden Welt in allen Zellen vorhanden sind. Sie weisen zwischen den verschiedenen Arten einen hohen Grad an struktureller und sequenzieller Homologie auf. Dadurch und dank ihrer funktionellen Autonomie sind zahlreiche Transposonen bei vollem Erhalt ihrer Transpositionsaktivität von einer Art auf andere Arten heterolog übertragbar. So springt das in Mais identifizierte Ac-Element nicht nur homolog in dieser Pflanze, sondern, nachdem es mit Hilfe der üblichen Genfähren dort eingetragen wurde, nachgewiesenermaßen auch heterolog in Tabak, Tomaten, Kartoffeln, Soja, Petunien, Arabidopsis. Konservierte Sequenzregionen pflanzlicher Transposonen finden sich u. a. auch in mobilen Elementen von Drosophila (P-Elemente, Hobo).

In den bisher untersuchten Genomen niederer und höherer Organismen sind Transposonen weit verbreitet. Dabei sind sie mehr oder weniger stabil integriert. Sie bilden Familien, die sich über zahlreiche Arten erstrecken können. Spezifisch ist nur das Transposon mit der Zahl seiner endständigen, umgekehrten Sequenzwiederholungen, nicht aber die Wirts-DNA.

Die Universalität der Transposonen äußert sich auch in dem ubiquitären Vorhandensein ihrer Strukturelemente in den Genomen aller untersuchten Organismen, z. B. mindestens 3 % der DNA in den 4 Chromosomenpaaren von Drosophila; ein Auftreten mit hoher Frequenz in den bis jetzt bekannten mittel- und hoch-repetitiven Sequenzen des Humangenoms, die einen bedeutenden Prozentsatz seiner gesamten DNA ausmachen.

### Klassifizierung und Eigenschaften

Transposonen zeigen eine hohe Zahl von Variationen und Übergangsformen.

Dies äußert sich zunächst in ihrem schematischen Aufbau:
1) Die einfachste Gruppe bilden die Insertions-Elemente (IS) mit maximal 2000 Basenpaaren (Bp) z. B. im Genom und in den Plasmiden der Bakterien. Ihr Aufbau entspricht dem Grundprinzip der Struktur eines Transposons: Transposase-Gen(e) flankiert von kurzen inversen terminalen Repetitionen. Diese beiden Mindestbestandteile sind für den Transpositionsmechanismus essentiell und dürfen genetisch nicht verändert sein.
2) In komplexen Transposonen sind zusätzlich eingefangene oder eingebrachte Gene bzw. Gen-Fragmente enthalten. Sie sind für den Vorgang der Transposition nicht essentiell und in dem Element beliebig ersetzbar. Sie benutzen das Transposon als Vehikel für ihre Wanderung im Genom. Dabei werden Eigenschaften, z. B. Resistenzen gegen Antibiotika und Schwermetalle übertragen (Tn bei Bakterien).
3) Bei zusammengesetzten Transposonen ist ein zentraler, kodierender Bereich von 2 Modulen der Gruppe 1 (IS-Elemente) flankiert. Auf diese Weise können ganze Gen-Gruppen transponiert werden.

Es gibt 3 Transpositionsmechanismen:
1) Bei der einfachen exzisiven Transposition wird der Platzwechsel durch ersatzloses Ausschneiden des Elements aus der Ursprungsstelle initiiert. Die untersuchten Familien pflanzlicher Transposonen folgen im wesentlichen diesem Mechanismus.
2) Bei der replikativen Transposition werden vor dem Platzwechsel Kopien des Transposons angefertigt. Im Endzustand enthält dann sowohl die Ursprungs- als auch die Zielstelle das Transposon. Dieser Mechanismus ist bei Bakterien weit verbreitet.
3) Bei der Retrotransposition entsteht als Zwischenstufe ein RNA-Transkript des Transposons, welches mit Hilfe reverser Transkriptase - ähnlich den Retroviren - in die Transposonen-DNA rückübersetzt und anschließend in die Ziel-DNA integriert wird.

Die Mechanismen 2 und 3 führen zwangsweise kumulativ zu einer Erhöhung der Transposonenzahl im Gesamtgenom. Bei Mechanismus 1 erfolgt die Transposomenmultiplikation wahlweise je nachdem, ob das Transposon während der Replikation zwischen bereits replizierten bzw. noch nicht replizierten DNA-Strängen springt. In jedem Falle erhöht sich auch hier im Laufe der Evolution die Zahl mehr oder weniger definitiv fixierten transponierbaren Elemente.

Es wird angenommen, daß ausgedehnte inaktive Genomabschnitte in Eukaryonten durch den retrotransposonalen Mechanismus entstanden sind. Der Informationsgehalt aller Eukaryonten ist nämlich ungefähr gleich: 50 000 - 100 000 kodierende Gene. Die enormen Unterschiede in den Genomengrößen der verschiedenen Arten resultieren aus der evolutionären Genomaufstockung mit Hilfe von Retrotransposonen. Sie ermöglichen das Längenwachstum und Strecken der Genome. Im Humangenom gehören die Lines-, Sines-, Alu-Familien dazu. Retrotransposonen werden stabil im Genom integriert und sind normalerweise nicht wieder excisierbar.

Die Transposition kann autonom erfolgen oder die Assistenz von Helferfaktoren erfordern. Es wird zwischen autonomen und defektiven transponierbaren Elementen unterschieden.
1) Autonome Elemente enthalten ein intaktes Transposase-Gen und unbeschädigte terminale Repetitionen. Sie sind voll funktionsfähig und können nachgewiesenermaßen heterolog in zahlreichen Organismen aktiv werden.
2) Defektive Elemente sind Transposonen, die ihre Autonomie verloren haben. Sie benötigen für ihre Genwanderung Helfertransposonen, z. B. ein autonomes Element der gleichen Familie, das für sie die Transposase kodiert. So ermöglicht das autonome Ac-Element im Mais den zahlreichen verschiedenen defektiven Ds-Elementen (einige 100) die Translokation. Derselbe Helfer-Effekt kann durch künstliche Expression einer geeigneten Transposase in der Wirtszelle erzielt werden.

An komplexen Transpositionsvorgängen ist ein Teil des Protein-Instrumentariums der eukariontischen Wirtszellen beteiligt: Mit Sicherheit nachgewiesen wurden die Rollen von Topo-Isomerase II und eines für die Bildung von Kruziformen verantwortlichen Hu-Proteins, z. B. der Hefe.

Schließlich können Transposonen auch nach der "Virulenz" ihrer Ausbreitung unterschieden werden.
1) Im Normalfall ist das Transposon im Genom stabiler oder instabiler Mutanten vorhanden, die mit anderen Mutanten und dem Wildtyp koexistieren.
2) Invasive Transposonen können sich wie durch Ansteckung schlagartig verbreiten und eine gesamte Artenpopulation "infizieren". So hat sich bei Drosophila das P-Element so virulent verbreitet, daß nur noch die Träger dieses Elements vorkommen. Der dieses Transposon nicht enthaltende ursprüngliche Wildtyp ist aus den natürlichen Populationen verdrängt worden. Das retrotransponierbare Element "Gypsy" von Drosophila ist ebenfalls hochinfektiös.

### Transposonen und Viren

Transposonen können mit "zellinternen Viren" verglichen werden. Mit den eigentlichen DNA-Viren gibt es begrenzte grundsätzliche Gemeinsamkeiten. Transposonen kodieren wie Viren zwar eigene Funktionsproteine (Transposasen), es werden aber keine Strukturproteine kodiert, da die Transposonen für ihre intrazellulare Translokation - im Gegensatz zu den Viren - kein Kapsid zu bilden haben. Transposonen sind Viren in permanant eklyptischer Phase.
- Gemeinsam mit den DNA-Viren haben die Transposonen die beschriebene hohe Zahl an Variationen und Übergangsformen im Hinblick auf Aufbau, Mechanismus, Funktion und Ausbreitung.
- Als direkte Berührungspunkte zwischen beiden genetischen Klassen gibt es Viren, die sowohl als Virus als auch als Transposon funktionieren können.
   Ein Beispiel dafür ist der virale Zyklus des Mu-Phagen mit wahlweise lytischer (mit Transposition) oder lysogener Zellinfektion (ohne Transposition). Die transpositionale Komponente löst hier die Aktivierung der im Wirtszellengenom ruhenden viralen Komponente aus.
   Ein weiteres Beispiel ist das Retrotransposon "Gypsy" von Drosophila. Es stellt gleichzeitig das erste bisher bekannte Retrovirus der Insekten dar. Es ist hoch infektös.
- Im allgemeinen gilt, daß Viren und Transposonen sich wechselseitig aktivieren können. Viren lösen transpositionale Aktivitäten aus, Transposonen aktivieren virale Zyklen.
- In ihrer primären Wirkung bringt die Insertion von Transposonen ähnlich der viralen Aktivität für die invadierte Wirtszelle und für den betroffenen Organismus zunächst nur Negativeffekte. Die Aktivität der besetzten Wirtsgene bzw. ihrer Promotoren wird herabreguliert oder total inhibiert. Dadurch werden Genwirkketten beeinträchtigt oder unterbrochen. Die entsprechenden Genprodukte werden nicht oder nur in unzureichender Menge hergestellt.
- Transposonen, die in nicht kodierenden Regionen des Genoms eingeparkt werden, sind zunächst funktionslos und verursachen, ähnlich manchen zellintegrierten DNA-Viren, keine gravierenden Störungen, jedoch belastet ihre stille Anwesenheit zumindest die Energiebilanz der Wirtszelle.

### Transpositionale Hemmeffekte

### Die Wirkung von Transposonen wird manifest, wenn

- das Transposon in exprimierenden Genen, in Promotoren oder in den Grenzcodierungsbereichen für Introns sitzt
- die Situation nach wenigstens einem generativen Zyklus von dem heterozygoten in den homozygot-blockierten Zustand übergeht.
   1) Durch die physische Anwesenheit des Transposons im kodierenden Bereich wird das betroffene offene Leseraster ("open reading frames" = ORF) gestört, die Anwesenheit in Introns kann den Spleißvorgang beeinträchtigen, die Anwesenheit im regulatorischen Bereich (z. B. Promotor) eines Gens kann dessen Expression beeinflussen. Als Folge werden Biosynthesen abgeschaltet, die Produktion normaler Transkripte wird blockiert. Dies äußert sich durch phänotypisch auffallende degenerative Effekte:
      - Bei Pflanzen bleiben beispielsweise Blütenfarbstoffe aus, Blattbezirke ohne Chlorophyll entstehen, Schrumpfungen an Früchten und Samen erscheinen, es kommt zu Stauchung und Zwergwuchs, Resistenzen brechen zusammen.
      - Bei Drosophila z. B. kommt es bei Besetzung der an der Expresion beteiligten Gene zur Ausbildung flügelloser Mutanten.
      - Das Transposon wird auf diese Weise durch äußere Merkmale sichtbar und quantifizierbar. Die transpositionale Inaktivierung von Genen kann zum Tod von Zelle, Organ und Individuum durch Dysfunktion vitaler metabolischer Vorgänge führen.
      - Die Lokalisation eines Transposons in den die Zellproliferation kontrollierenden Genen kann Tumorbildungen oder apoptotische Selbstauslöschung auslösen. Umgekeht kann eine Transposonen-Insertion solche Prozesse auch zum Stillstand bringen.
         Verläßt das Transposon das besetzte Gen, kann die entsprechende Revertante den Wildtypcharakter ganz oder teilweise wiedererlangen. Aus dem Wechselspiel zwischen Insertion/Excision erwächst die für die Transposonen typische Instabilität der Mutation.
   2) Ist das Transposon in den umfangreichen nicht kodierenden Bereichen eukariontischer Genome lokalisiert, bleiben die transpositionalen Hemmeffekte zunächst meist inapparent. Das Transposon sitzt symptomlos im Genom ähnlich den retroviralen Proviren und anderen persistent genomintegrierten DNA-Tumorviren.

### Leistungsabfall bei Hochzuchtsorten

Bei auf bestimmte Nutzeigenschaften hochgezüchteten Arten und Sorten kann es zu verstärkter Bildung und kumulativer Ansammlung von Transposonen kommen (Transposonenüberladung). Dies zeigt sich beispielsweise am Mais, der wichtigsten und am besten hochgezüchteten Nutzpflanze des Menschen: Hier wurden zuerst und in größerer Zahl die phänotypisch auffallenden Effekte ganzer Transposonenfamilien entdeckt (Ac/Ds; En/Spm, Mu). Ausführlich wurden bisher die transpositionalen Hemmeffekte auf diejenigen Genwirkketten untersucht, deren Produkte visuell besonders auffällig sind: Anthocyane, Flavone, Chlorophyll und Stärke.

Der Züchtungsvorgang führt zu einer unterschiedlichen Behandlung zwischen solchen Transposonen, die sich in exprimierenden Genen anreichern und so veränderte Merkmale ausprägen und solchen, die sich ohne sichtbaren Effekt in nicht kodierenden Bereichen akkumulieren.

Selektion und Züchtung werden nach den vom Züchter gewünschten sichtbaren Merkmalen gesteuert und betreffen nur die Transposonen aus dem exprimierenden Bereich. Negative Merkmalsausprägungen werden zusammen mit den sie verursachenden Transposonen eliminiert, erhalten bleiben die "unsichtbar" integrierten Transposonen. In der Tat ist der nicht kodierende Genbereich vom züchterischen Selektionsdruck zunächst nicht betroffen, die Transposonen können dort ohne Kontrolle durch die Selektion akkummuliert werden. Mit züchterischer Entfernung vom Wildtyp kann die Transposonenansammlung in stillen Genombereichen wachsen. Im Epidemiefall kann es sich jedoch erweisen, daß man mit der Selektion auf gewünschte Leistungsmerkmale die Anfälligkeit gegen bestimmte Infektionen negativ beeinflußt hat, d. h. die Krankheit wurde mitgezüchtet:
- So entwickeln Wildpflanzen nach Viroidinfektionen kaum Symptome, während hochgezüchtete Monokulturen, Plantagen- und Gewächshauspflanzen empfindlich reagieren.
- Das CRP-Virus ("Cottontail Rabbit Pappiloma Virus") verursacht in Zuchtkaninchen maligne Tumore, während es für Wildkaninchen weitgehend inoffensiv bleibt.
   Insgesamt betrachtet können Transposonen sowohl direkt sichtbare als auch verzögernd auftretende, schwerwiegende Negativeffekte verursachen.

Eine regulierbare, streßfreie Aktivierung von Transposonen in einem intakt bleibenden Genom ist in vielfacher Hinsicht von enormer wirtschaftlicher Bedeutung.

### Nutzeffekte

Wegen der negativen Primäreffekte stellt die Transposition eine potentielle Bedrohung für die Stabilität der Arten dar. In natürlichen Populationen wird daher die Transpositionsrate von der Zelle streng kontrolliert und extrem niedrig gehalten: Pro Zellgeneration finden nur 10⁻⁷ bis 10⁻⁴ solcher Ereignisse satt.

Umgekehrt beweist das universelle Vorkommen der Transposonen ihre grundsätzliche Notwendigkeit:

Die ständige und zwischen den Arten synchronisierte Neukombination von Genelementen durch Transposition ermöglicht und generiert die Diversität der DNA. Das Transposon ist der Motor der Evolution zu rasch und stets besser angepaßten höheren Organisationsformen des Lebens.

Es wird angenommen, daß Transposonen während des Evolutionsprozesses die horizontale Übertragung genetischer Information sowohl zwischen verwandten als auch zwischen weit entfernten Arten, z. B. Pflanze und Tier vermitteln. Transposonen würden auf diese Weise die Evolution durch gegenseitige genetische Abstimmung der Lebewesen synchronisieren (konvergierende Evolution).

Als Gegenspieler zur genetischen Starre der Replikation sorgt die durch die Transposition generierte genetische Diversität bei abrupten Umweltveränderungen für die Erhaltung der Art durch das selektive Überleben der jeweils bestangepaßten Individuen aus einem breiten Spektrum von Mutanten.

Ein Beispiel für eine schnelle, umweltbedingte Anpassung ist die transpositionsbedingte Resistenz von Mikroorganismen gegen Antibiotika und Schwermetalle. In diesen Fällen reichern sich Resistenzgene mit Hilfe der sie vehikulierenden Transposonen im Genom und in Plasmiden an. Aufgrund der Universalität von Transposonen ist die Übertragung der Informationen nicht auf verwandte Spezies beschränkt und kann sich rasch zu einem allgemeinen Anpassungsphänomen entwickeln, das von einer großen Anzahl von Arten verwendet wird. Transpositionsbedingte genetische Veränderungen werden auf diese Weise zu einem Instrument der Eigenerhaltung für ganze Artenfamilien, die auf diese Weise abrupt veränderte Umweltbedingungen überleben, aber auch zu einem Mittel des Selektionskampfes zwischen den Subspezies (z. B. mittels "Killerproteinen") einer Art.

Durch Insertion von Transposonen kann es auch zu Aktivierung von Genen der Wirtszelle kommen. Ein transposoneneigener Promotor liest über die Eigenfunktionsgene des integrierten Transposons hinaus in die Wirtszellen-DNA hinein und aktiviert somit angrenzende Gene aus dem Wirtsbereich, die normalerweise nicht oder kaum exprimiert werden.

### Transposonen als genetische Werkzeuge

In letzter Konsequenz ist das Transposon das streng kontrollierte genetische Werkzeug der Evolution, die weder explodieren noch erstarren darf.

Als ein solches Werkzeug ist es auch in der gentechnischen Forschung und Züchtung prinzipiell anwendbar.

Die Aufgabe besteht hier in der gezielten Suche nach einzelnen Genen in den immens langen Nukleotidsträngen der Chromosomen. Die selektive Gensuche ist von besonderer Bedeutung, weil sie eine wirtschaftlich akzeptierbare Alternative zu anderen Methoden der Genisolierung einschließlich der Totalsequenzierung von Gesamtgenomen darstellt. Letztere würde bei 1,5 Millionen eukariontischen Arten und einer mittleren Genomgröße von ca. 10⁹ Basenpaaren eine nicht realisierbare astronomische Arbeitsleistung erfordern (sequenzielle Identifizierung von 10¹⁵ Basenpaaren).

Mit Hilfe eines Transposons kann ein gesuchtes Gen (erkennbar an der instabilen Ausprägung des betreffenden Gens) etikettiert und, wenn die Nukleotidsequenz des Transposons bekannt ist, auch kloniert werden. Eine vorherige Kenntnis der primären Funktion des betreffenden Gens bzw. eine Kenntnis des entsprechenden Genprodukts ist dafür nicht erforderlich. Das einzige rekognitive Kriterium ist Anwesenheit des Transposons sowie seine Nukleotidsequenz. Das Verfahren wird als "gene tagging" bzw. "transposon tagging" bezeichnet und konnte bereits mit Erfolg eingesetzt werden.

Das "transposon tagging" ist damit die Methode der Wahl zur selektiven Klonierung beispielsweise von Resistenzgenen und Genen mit regulatorischen Funktionen sowie zur genomischen Defektsuche bei genetischen Krankheiten.

So wurden mit dieser Methode zunächst im Mais und im Löwenmaul eine Reihe von Strukturgenen und insbesondere von Genen mit regulatorischer Funktion erkannt und kloniert und zwar mit Hilfe der in diesen Pflanzen natürlicherweise vorhandenen und gut untersuchten Transposonen (z.B. Ac/Ds, En/Spm und Mu im Mais bzw. Tam 1 und Tam 3 im Löwenmaul).

Inzwischen ist es auch gelungen, einige der bekannten Transposonen des Mais und des Löwenmauls mit Hilfe geeigneter Genfähren in andere Pflanzenarten wie beispielsweise Tomaten, Kartoffeln, Petunien und Arabidopsis zu übertragen. Interessanterweise können sie auch im Genom dieser heterologen Systeme ihre Position wechseln und instabile Mutationen erzeugen. Dadurch ist das selektive Markieren und Klonieren von Genen auch in Pflanzenarten möglich geworden, die natürlicherweise keine bzw. keine dafür geeigneten Transposonen besitzen. Prinzipiell gesehen sind das "gene tagging" und damit die selektive Gensuche mittels Transposonen zu einer universell einsetzbaren Methode geworden.

Erfolg oder Mißerfolg des "gene taggings" hängen allerding in homologen wie in heterologen Systemen von der Transpositionsrate des verwendeten Transposons ab, die natürlicherweise extrem niedrig ist (ca. 1 : 10⁶).

In manchen Fällen wäre es vorteilhaft, das gewünschte Gen simultan durch 2 unterschiedliche Transposonen zu kennzeichnen. Dies stellt ein noch viel selteneres Ereignis dar. Das Warten auf ein statistisches Erfolgsereignis wird unerträglich lang.

Eine weitere Minderung der transpositionalen Erfolgswahrscheinlichkeit ergibt sich daraus, daß die an sich schon unwahrscheinliche Freigabe des Transposons mit der Integration in ein Ziel-Gen beendet werden soll, welches nur eines unter üblicherweise 100 000 eukariontischen Genen ist.

Das für die Etikettierung verwendete Transposon muß sich bei seiner Transposition gerade in dieses Gen integrieren. Die theoretisch konzipierte einmalige Methode der gewünschten Etikettierung eines bestimmten Gens war durch das bisher unüberwindliche Hindernis des Fehlens einer störungsfreien Aktivierung der Transposition kaum realisierbar (statistische Wahrscheinlichkeit ca. 1 von 10¹⁰ Individuen).

So mußten z. B. bei Pflanzen eine sehr hohe Zahl von Individuen angebaut und auf den Mutanten-Phänotyp hin untersucht werden.

Trotz großer Anstrengungen konnten auf diese Weise bisher nur einige wenige wirtschaftlich wichtige Gene kloniert werden und dies unter ungeheurem Aufwand an Material, Zeit und Kosten.

Die Beseitigung dieses Hindernisses durch eine signifikante Erhöhung der Transpositionsrate ist deshalb für das "gene tagging" von enormer Bedeutung.

### Erfindungsgegenstand und Ausführungsbeispiele

Der Erfindungsgegenstand besteht in der überraschenden, dramatischen Erhöhung der Transposonenmobilität (Excision und Integration). Der Effekt wird streß- und zerstörungsfrei durch die Einwirkung bio-assimilierbarer Borverbindungen erzielt. Der Erfindungsgegenstand wurde am Beispiel der Vertreter zweier bekannter und weitverbreiteter Transposonenfamilien (TAM 1 und TAM 3) systematisch untersucht und wird anhand der nachfolgenden Ausführungsbeispiele belegt. Durch die Wahl dieser beiden Pilotfamilien werden die wichtigsten bis jetzt experimentell untersuchten Transpositionssysteme in die erfinderische Verbesserung einbezogen. Auf dieser Grundlage kann erwartet werden, daß die Boraktivierung transposabler Elemente auch für alle anderen Systeme universell Gültigkeit hat.

Das Transposon TAM 1 besteht aus 15164 Basen (B) und beeinflußt bei der hier verwendeten Mutante von Antirrhinum majus das niv-Gen, wo es beispielsweise 17 Basenpaare (Bp) entfernt von der TATA-Box des für die Chalkonsynthase kodierenden Gens integriert ist. Es wird von jeweils 2 perfekt invertierten Wiederholungssequenzen aus 13 Basen flankiert und erzeugt am Integrationsort eine 3 Basenpaare lange Sequenzverdopplung ("target site duplication" = TSD). TAM 1 kodiert zwei Transposasen. Aufgrund dieser kompletten Eigen-Ausrüstung besitzt es eine perfekte Autonomie und kann - heterolog übertragen - auch in anderen Pflanzen springen.

Zur gleichen Familie gehören das transponierbare Element En/Spm im Mais, Tpm 1 in der Sojabohne und zahlreiche andere, die heterolog in andere Pflanzengenome implantiert und dort aktiv werden können. Bei der strangversetzten Excision hinterläßt das ausgeschnittene Transposon Sequenzduplikationen in Form von zusätzlichen Basenpaaren ("footprints") im zurückbleibenden und wieder ligierten Genabschnitt. Auf diese Weise ist der Excisionsort markiert. Das Gen der durch die Excision entstandenen Revertante ist nur selten (10 %) mit dem des Wildtyps identisch. Durch Punktmutationen kann das Gen-Produkt verändert werden. Sowohl die Transposition selbst als auch die verbleibenden Sequenzveränderungen am Excisions- und am Integrationsort stellen Glieder in einer Kette von neugebildeten stabilen oder instabilen Mutanten dar. Transpositionen erzeugen ein ganzes Spektrum von Mutanten.

Das Transposon TAM 3 - ebenfalls im Chalkon-Synthase-Gen von Antirrhinum lokalisiert - besteht aus 3629 Bp, besitzt eine endständige perfekt invertierte Sequenzwiederholung von 11 Basen und erzeugt eine 8 Basen lange "target site duplication" (TSD). Die Translokation wird autonom von einer einzigen selbstkodierten Transposase gesteuert. TAM 3 ist repräsentativ für eine weitreichende Familie mit gleichen oder ähnlichen Transpositionsparametern (Elemente Ac/Ds im Mais, Hobo in Drosophila).

TAM 1 und TAM 3 sind bei den verwendeten instabilen Mutanten von Antirrhinum majus in dem die Chalkonsynthase (Schlüsselenzym) kodierenden Gen lokalisiert. Dadurch wird die Chalkonsynthese herabreguliert bzw. blockiert. Chalkone sind die Grundkörper für die Biosynthese der weitverbreiteten Stoffklasse der Flavonoide, u.a. auch der Anthocyane, die im Antirrhinum in Blüten und Blättern charakteristische Rotfärbungen hervorrufen.

Die Mobilität der Transposonen TAM 1 und TAM 3 ist in diesen Mutanten von Antirrhinum direkt mit dem Anthocyangehalt und den Färbungen in Blüten und Blättern der Pflanzen korreliert. Diese stellen die beobachtbaren und meßbaren Parameter für die Transposonenaktivität dar:
- Den unbewegt im Chalkon-Synthase-Gen fixierten Transposonen TAM 1 oder TAM 3 entsprechen ungefärbte Blüten und niedrige Anthocyangehalte in allen Teilen der Pflanze.
- Der Excisionsvorgang erlaubt in den meisten Fällen die Reaktivierung der Genwirkkette zur Chalkonbildung, restituiert die Anthocyansynthese und manifestiert sich durch das Wiedererscheinen rotgefärbter Bezirke im Blütengewebe. Frühe Excisionsereignisse werden auf nachfolgende Zellgenerationen weitergegeben und erzeugen größere Farbbezirke; spätere Excisionen werden durch immer kleinere Tüpfelungen sichtbar. Auf diese Weise konnte die Transposition qualitativ (ja/nein), quantitativ (wie oft) und zellgenerationsdynamisch (wann) verfolgt werden.
- Wegen der extrem niedrigen Transposonenaktivität (normalerweise: 1 : 10⁻⁶ pro Zellgeneration) sind die Blüten der Antirrhinum-Mutanten von TAM 1 bzw. TAM 3 weiß mit gelegentlichen Farbtüpfelungen.

Erfindungsgemäß wird die natürlicherweise extrem niedrige Transposonenaktivität durch Zufuhr geeigneter Mengen von bioassimilierbaren Borverbindungen um Zehnerpotenzen angehoben.

Dies zeigt sich in dramatischer Weise in dem verwendeten Experimentalsystem wie folgt:
- Je nach Bormenge und Applikationszeitpunkt und -form wird eine bis zu totale Durchfärbung der Blüten beobachtet.
- Der Anthocyangehalt des Blütengewebes steigt größenordnungsmäßig um den Faktor 5 an.
- Der gleiche Effekt wird in der unteren Epidermis der jüngeren Blätter beobachtet. Diese sind grün bei Anwesenheit des Transposons (TAM 1 bzw. TAM 3 im Chalkon-Synthase-Gen). Durch natürliche Excision treten äußerst selten rote Bereiche auf, die, analog zu den Blüten, bei Anwendung von Borverbindungen dramatisch verstärkt werden.

Die Applikation von Borverbindungen bewirkt also mittelbar oder unmittelbar eine Revertierung der transposonen-beladenen, instabilen Mutanten in ein Spektrum wildtyp-ähnlicher Formen.

Damit zeigt sich in unerwarteter Weise die genetische Wirksamkeit einer Verbindungsklasse, die in der Biochemie über einen großen Zeitraum vernachlässigt wurde:

Wegen des Mangels an einem geeigneten Radio-Isotope (längstlebiges Isotop T_{1/2}= 0,8 sec.) ist das Bor in der innovativen Forschung in die Rolle des "vergessenen Elementes" gedrängt worden. Deswegen ist es auch bei molekularbiologischen Untersuchungen nicht eingesetzt worden. In unserem Falle erfolgten die Identifizierung und die Bestimmung von Borverbindungen durch erst kürzlich entwickelte Techniken: Atomabsorptions-Spektrometrie (ICP-Technik) und NMR-Spektroskopie von ¹¹Bor.

Erschwerend hinzu kommt die chemische Nichtfaßbarkeit der Borverbindungen in der lebenden Zelle, wo eine Vielzahl sich rasch ineinander umwandelnder Komplexe und Verbindungen koexistieren. Die zu investierende chemische Arbeitsleistung gleicht der Untersuchung der Huminstoffsysteme, die trotz ihrer universalen Wichtigkeit bis heute noch nicht gelungen ist.

In einer beispielhaften Ausführungsform zur Stimulierung der Transposonenaktivität durch bioassimilierbare Borverbindungen wurde wie folgt verfahren:

Einige hundert Samenkörner der instabilen Antirrhinum-Mutanten (TAM 1 und TAM 3) wurden jeweils unter üblichen Gewächshausbedingungen auf Blähton oder anderen inerten Substraten zur Keimung gebracht und auf 5 cm Länge angezogen.

Kontroll- und Versuchspflanzen wurden dabei mit Hoaghland-Nährlösungen behandelt, die letzteren wurden je nach Versuchsreihe mit 2 bis 20 ppm Bor (z. B. als Borsäure) supplementiert. Danach wurden die Jungpflanzen in Hydrokulturtöpfen unter streng kontrollierten Bedingungen und bei regelmäßiger Erneuerung der Nährlösung bis zur Blüte weitergezogen. Dies geschah je nach Versuchsserie bei verschiedenen Borkonzentrationen (0,5 bis 30 ppm Bor).

Auch der Zeitpunkt der ersten Bor-Supplementierung wurde variiert: Bei einigen Versuchen geschah die Borzugabe in späteren Entwicklungsstadien der Pflanze, bei anderen bereits von der Keimung an.

Die Borapplikation kann zusätzlich oder allein auch folial erfolgen, vorzugsweise unter Zufügung eines Benetzungsmittels. Folial ist es möglich, auch partiell zu applizieren und den Effekt zeitweilig auf einen Teil der Pflanze zu beschränken.

Die Auswertung erfolgte optisch halb-quantitativ durch Analyse der Färb- und Tüpfelungsmuster der Blüten sowie nach Extraktion spektrophotometrisch durch Bestimmung der Anthocyanmengen in Blüten und Blättern. Extrahiert wurde in bekannter Weise mit 1 % HCl in Methanol, gefolgt von einer HPLC Trennung (C-18 Säule, 5 % Ameisensäure in Acetonitril) und optischer Detektion bei 530 nm.

Dabei ergab sich beispielsweise:
- Erfindungsgemäß borbehandelte Mutanten zeigten parallel zur applizierten Bormenge und zur Dauer der Boreinwirkung progressiv flächendeckende Rotfärbungen. Frühe Borapplizierung führte zu frühen Transformationsereignissen mit großen Anthocyan-Bereichen. Aus späterer Behandlung resultieren kleine Farbflecken, deren Zahl mit wachsender Bormenge bis zur partiellen Durchfärbung zunahm. Verbliebene weiße Sprenkelungen sind z. T. auf Footprint-Mutanten zurückzuführen, bei denen zwar das Transposon entfernt, das befreite Gen aber dabei stabil punktmutiert wurde und seine Kodierungsfähigkeit irreversibel verloren hat.
- Dagegen zeigten die Kontrollpflanzen der instabilen TAM 1- und TAM 3-Mutanten in unüblicher Weise rote Sprenkelungen auf weißem Grund.

Noch überzeugender war die physiko-chemische Anthocyan-Bestimmung:
- Der Farbstoffgehalt in den Blüten der borbehandelten Pflanzen war bis zu 5 mal höher als in den Kontrollpflanzen.
- Besonders ausgeprägt war der Unterschied in den aus den Blättern isolierten Anthocyanmengen: Pflanzen mit hoher und langandauernder Boreinwirkung zeigten intensiv rot gefärbte Anthocyanablagerungen in der Epidermis der Blattunterseiten, was bei den Blättern der Kontrollpflanzen nicht der Fall war. Physiko-chemisch konnte bei letzteren kein Antocyan nachgewiesen werden, während bei den Borpflanzen bedeutsame Mengen isoliert und identifiziert wurden.

Diese Befunde belegen die erfindungsgemäße bedeutsame Stimulierung der Transposonenaktivität bis hin zur vollständigen Entfernung aller Transposonen aus dem Ursprungslocus.

Gleichzeitig bewirkt die erfindungsgemäße Transpositionsbeschleunigung das rasche Auftreten eines breiten Spektrums neuer stabiler Mutanten, was einer Evolution im Zeitraffer-Tempo gleichkommt.

Ursache dazu sind die oben beschriebenen Mechanismen, insbesondere Footprint-Mutationen im Ursprungs-Gen und "target-site-duplications" (TSD) im Ziel-Gen, die sich den durch die eigentliche Genwanderung bewirkten Effekten überlagern. Auch das Transposon selbst kann bei der Wanderung mutieren (z. B. Deletionen, Richtungsinversion von Genen und/oder Promotern).

Die durch die Borbehandlung verursachte Mutationsbeschleunigung zeigt sich ansatzmäßig bereits in der Einzelpflanze durch das Auftreten verschiedener Transcriptionsvarianten in den Genprodukten. So kam es erfindungsgemäß bei den aus der Borbehandlung resultierenden Antirrhinum-Revertanten von TAM 1 bereits in der gleichen Pflanze zur Bildung footprintbedingter Varianten der Chalkon-Synthase-Expression.

In allgemeiner Weise kann die Applikation der borhaltigen Lösungen nutritiv über Böden oder in Hydrokultur oder folial durch Versprühen oder über beide Wege erfolgen. Den Pflanzen werden im Boden oder sonstigen Nährmedien und/oder auf jede andere Weise bioassimilierbare, anorganische oder organische Borverbindungen im Rahmen der für die Spezies und Sorte geltenden toleranzgrenzen zugeführt, wobei die Applikationsform jede Art von boden-, planzen- und allgemein bioverträglichen anorganischen und organischen Borverbindungen doer Mischungen daraus sein kann, vorzugsweise wäßrige Lösungen von Borsäure, von Borarten, die ggf. neutralisiert werden, von organischen Borverbindungen, insbesondere von Boratchelatkomplexen mit Polyolen wie Glykol, Glycerin, Mannit oder Sorbit, mit Oxycarbonsäuren, wie Milch-, Wein- oder Zitronensäure oder in Form von Kohlenhydratkomplexen mit Glukose, Galaktose, Fruktose, Maltose, Laktose oder jeder anderen Art von Mono-, Di-, Tri- und Oligosacchsariden als glykosidische Liganden.

Zur Stimulierung der Transposonenmobilität bei Zellkulturen in der Mikrobiologie sowie im Veterinär- und Humanbereich finden im wesentlichen die gleichen Applikationsformen unter strenger Beachtung der jeweiligen Toleranzgrenzen Verwendung, ggf. wird die Wahl von der Anpassung an bestehende Puffersysteme mit bestimmt.

Wegen der Universalität der Transposition ist auch das erfindungsgemäße Monitoring der Transposonen in seinem Anwendungsbereich universell. Die dosierbare, zerstörungsfreie Freigabe der natürlicherweise genetisch streng versiegelten Transposonen löst eine ganze Serie von Anwendungen mit großem wirtschaftlichen Interesse aus. Sie erlaubt die Realisierung einer Vielzahl von Prozessen auf den Gebieten der Züchtung, Genetik, Therapie u. a., die bisher zwar theoretisch möglich, wegen der natürlicherweise extrem niedrigen Transpositionsrate jedoch nicht oder nur unter ungeheurem Aufwand durchführbar waren. Dies wird an weiteren Anwendungsbeispielen beschrieben.

Die neuen folgenden Beispiele erläutern die Verwendung von Bor näher.
1. Entranspositionierung zwecks Sortenverbesserung
   Ähnlich wie bei den untersuchten Mutantenlinien von Antirrhinum werden auch bei anderen Pflanzen und Sorten funktionshemmende Transposonen aus wirtschaftlich wichtigen Genen zerstörungsfrei entfernt, wenn die Pflanzen der erfindungsgemäßen Borbehandlung unterworfen werden. Zum Beispiel werden auf diese Weise bei der Sortenzüchtung Transposonen von einer hochstörenden Position in unschädliche Genomstellen weggeparkt. Die bis dahin durch die Anwesenheit des Transposons gehemmte Genfunktion (Resistenzen gegen Parasiten, Umweltgifte oder eine sonstige positive Eigenschaft, Produktion gewünschter Pflanzeninhaltsstoffe wird auf diese Weise gesteuert. Die erfindungsgemäße Borbehandlung von Pflanzen ist überall dort angetan, wo im Laufe eines Züchtungsvorganges Transposonen ungewollt und unbeachtet angesammelt wurden. Durch die Beseitigung der transpositionalen Hemmung werden Sorten von spezifischen Mängeln befreit, die während der Züchtung miterworben worden.
   Durch die erfindungsgemäße Borapplikation können in Sorten vorhandene spezifische Mängel behoben werden.
2. Früherkennung von Sorteninstabilitäten
   Durch die erfindungsgemäße erhöhte Borzugabe werden transposonenbedingte Instabilitäten in Sorten im Zeiraffertempo aufgespürt.
   Der normale Prüfungsgang z. B. einer Pflanzensorte ist ein aufwendiger und zeitraubender Vorgang der sich über zahlreiche Jahre, Boden- und Klimazonen erstreckt. Bor erlaubt die frühzeitige Erkennung eventuell vorhandener Transposonen, die sich negativ auf die Stabilität einer Sorte auswirken können. Transposonenbedingte Mängel und Abbauerscheinungen, die natürlicherweise irgendwann im Laufe einer Streßsituation auftreten und die die Qualität einer Sorte infrage stellen, werden frühzeitig und beschleunigt aufgespürt. Dem normalen Prüfungsgang vorgeschaltet werden durch diese Bordiagnose Fehlinvestitionen in der Arten- und Sortenzüchtung vermieden.
   Bor erlaubt auf diese Weise die Früherkennung von Sorteninstabilitäten.
3. Auffinden neuer Transposonen
   Bis jetzt sind nur wenige Transposonen bekannt. Zum Beispiel sind in vielen Pflanzen noch keine natürlicherweise darin vorhandenen aktiven Transposonen gefunden worden. Will man Transposonen als genetische Werkzeuge verwenden, müssen Fremdtransposonen heterolog mittels Genfähren in das Genom eingeführt werden. Durch die erfindungsgemäße Mobilisierung eventuell natürlich in der entsprechenden Art vorhandener Transposon werden diese erkannt und für die beschriebenen Anwendungsbereiche verfügbar. Die homologe Verwendung von Eigentransposonen ist in vielen Fällen einfacher, sicherer und kostengünstiger als ihre heterologe Insertion in das gewünschte Artengenom.
   Bor erlaubt die Erkennung und Verwendung von Faktoren, die instabile Mutationen verursachen.
4. Beschleunigung der Mutation als gewünschter Effekt
   Durch die erfindungsgemäß beschleunigte Transposonenbewegung wird die Mutantenzahl dramatisch erhöht.
   Jede Excision und Integration eines Transposons ist mit Sequenzduplikationen im Wirtsgenom verbunden ("footprints" bzw. "target site duplications"), die ganz, teilweise oder nicht erhalten bleiben.
   Auch das aktivierte Transposon kann mutieren. Dies bewirkt eine Kaskade genetischer Veränderungen.
   Die erhöhte Borzugabe führt zu schnelleren und breiter gefächerten Mutationen, aus der die Individuen die Träger der gewünschten Verbesserung sind, rascher und besser selektioniert werden können.
   Die Erfindung stellt ein Verfahren zur beschleunigten Erlangung neuer pflanzlicher, tierischer und mikrobiologischer Sorten dar.
5. "gene tagging" als ökonomischer Prozeß
   Die Verfügbarkeit wirtschaftlich wichtiger Gene ist ein allgemeines Bedürfnis auf den Gebieten Züchtung, Genetik und Therapie. Zahlreiche prinzipiell mögliche Verfahren (z. B. somatische Gentherapie) waren nicht ausführbar, weil die implizierten Gene nicht charakterisiert sind.
   Durch die bor-induzierte Erhöhung der Transposonenmobilität wird das selektive Auffinden von Genen mit Hilfe von "gene tagging" um Zehnerpotenzen rentabler, rascher und billiger.
   Beispielsweise wird dadurch die systematische Suche nach Genen für Resistenzen gegen biotischen und abbiotischen Streß, für die pharmazeutisch gewünschte, gezielte Produktion aller Art von Metaboliten und für die Verbesserung der inneren und äußeren Qualität landwirtschaftlicher und aller anderen Arten biologischer Produkte möglich
6. "gene tagging" in Gewebekulturen
   Die erfindungsgemäße hohe Effizienz der Transposition rückt auch ihre Anwendung in Kulturen tierischer und menschlicher Zellen in den Bereich der Möglichkeiten. Im Humanbereich können durch effizientes "gene tagging" Ursachen von und Therapien für Krankheiten gefunden werden, die genetisch (mit)bedingt sind. Etwa 1000 humane Erbkrankheiten sind bisher genetisch identifiziert. Der Großteil ihrer ständig steigenden Zahl ist unaufgeklärt. Jeder Mensch trägt ca. ½ Dutzend schwerwiegend genetischer Deffekte mit sich. Borinduziertes "gene tagging" erlaubt die Entwicklung rascher Routinetestmethoden.
7. Gentransfer mit Hilfe boraktivierter Transposonen
   Bei genügend hoher Transpositionsfrequenz können Transposonen in effizienter Weise als Genfähren verwendet werden. Bei den bisherigen Methoden erfolgt der Gentransfer weitgehend unspezifisch unter Bildung einer Vielzahl nicht vorhersehbarer stabiler und instabiler Mutanten. Bei der Verwendung von boraktivierten Transposonen, die mit einem zu transportierenden Gen beladen sind, läßt man das Transposon so lange springen, bis es an der gewünschten Stelle angekommen ist. Auf diese Weise kann ein in einer anderen Sorte gefundenes Resistenzgen in das Genom einer Hochleistungssorte implantiert werden, der die von diesem Gen kodierte Eigenschaft fehlt.
8. Gezielte Aktivierung von Genen (Transposon als Transkativator)
   Ein Transposon mit einem transposoneneigenen, starken Promoter wird in der Nähe und in der richtigen Leserichtung eines normalerweise schwach oder nicht exprimierten oder durch pathogene Vorgänge inaktivierten Wirtsgenes insertiert. Der transposoneneigene Promoter liest über die Eigenfunktionsgene hinaus und (re)aktiviert das anvisierte Wirtszellengen. Voraussetzung dafür ist eine erhöhte Transpositionsrate, die wieder erfindungsgemäß durch Bor erzielt wird.
9. Gezielte Inaktivierung von Genen
   Unerwünschte Genexpressionen werden erfindungsgemäß durch gezielte Inplantation von Transposonen unterdrückt. Voraussetzung dazu ist, daß die Transposonen in genügender Anzahl zur Verfügung stehen, was durch eine hohe Mobilität erreicht wird.
   Von besonderer Bedeutung ist dabei die selektive Unterbrechung viraler Zyklen und der Proliferation tumoraler Zellen sowie die Expression von Proteinen die apoptotische Selbstauslöschung der Zellen auslösen ("Transpsonentherapie").
   In einer speziellen Ausführungsform wird die Konstruktion und Verwendung von deffekten transposablen Elementen (DTE) vorgeschlagen. Diese sind zur autonomen Transposition unfähig und üben in der normalen Zelle (mit oder ohne Bor) keine Hemmfunktionen aus. Ihre Aktivität ist von zellulären Hilfsfunktionen in der Weise abhängig, daß sie beispielsweise nur in virusinfizierten Zellen aktiv werden können. Bor leistet hier die erfindungsgemäße Excisionshilfe, die zur Erlangung einer genügend hohen Zahl hemmender Integrationsereignisse notwendig ist.

Zur selektiven Inhibierung retroviraler Zyklen, z. B. bei Leukämie- und HIV-Viren, wird in einer besonderen Ausführungsform die Verwendung erfindungsgemäß boraktivierter defektiver retrotransponierbarer Elemente (DRTE) vorgeschlagen.

In derartigen, helferabhängigen Elementen ist das offene Leserraster (DRF) zur Expression der für die Retrotranspostion notwendigen reversen Transkriptase (RT) defekt.
- Um überhaupt zu springen, benötigt das Element eine transposonenexterne Helferfunktion. Diese wird vom Gen Pol des zu bekämpfenden Virus geliefert, das in den infizierten Zellen eine große Menge reserve Transkriptase (RT) erzeugt.
- Um oft zu springen und therapeutisch effizient zu sein, benötigt das Element die erfindungsgemäße Aktivierung durch Borverbindungen. In infizierten Zellen springt das gen-therapeutische Transposon solange, bis
   a) der virale Zyklus durch Transposoneninsertion inhibiert ist
   b) die infizierte Zelle durch übermäßige Chromosomenauflockerung abstirbt und keine Viren mehr erzeugen kann.

Gesunde Zellen werden davon nicht betroffen, da das defektive Transposon nicht über die notwendige Menge an Retrotransposase (RT) verfügt und virusabhängig ist.

Während z. B. die bisherigen Anti-HIV-Strategien hauptsächlich auf die Suppression der reversen Transkriptase (RT) hinausliefen, was schlechthin unmöglich erscheint, da wahrscheinlich die meisten Zellgenom zumindest einige Moleküle RT als evolutionäres Relikt mitcodieren, wird im Gegensatz dazu die RT in diesem System zur Abwehr des sie erzeugenden Virus benutzt: je mehr Virus, desto mehr RT, desto mehr erfindungsgemäß borstimulierte Transpositionsereignisse, desto effizienter die Virusabwehr durch immer häufigere Transposonensprünge.

Das Virus leitet auf diese Weise seine selektive Selbstzerstörung ein.

## Patentansprüche

1. Verwendung von Bor in Form von bioassimilierbaren Borverbindungen zur Regulierung der genetischen Transposition bei Pflanzen, Tieren, deren Zellkulturen und bei Mikroorganismen.

2. Verwendung von Bor nach Anspruch 1, wobei mit Hilfe der Regulierung der genetischen Transposition bei Pflanzen und deren Sorten vorhandene spezifische Mängel behoben werden.

3. Verwendung von Bor nach Anspruch 1 zur Früherkennung von Sorteninstabilitäten.

4. Verwendung von Bor nach Anspruch 1, wobei die Regulierung der genetischen Transposition zur Erkennung von Faktoren, welche instabile Mutationen verursachen, ausgenutzt wird.

5. Verwendung von Bor nach Anspruch 1 zur Beschleunigung der Züchtung neuer Sorten.

6. Verwendung von Bor nach Anspruch 1 zum gene tagging.

7. Verwendung von Bor nach Anspruch 1 zum Gentransfer.

8. Verwendung von Bor nach Anspruch 1 zur Aktivierung von Genen.

9. Verwendung von Bor nach Anspruch 1 zur Inaktivierung von Genen.
